# EUROPEAN PATENT APPLICATION

(11) **EP 1 293 186 A2**
(43) Date of publication of application: **19.03.2003**
(21) Application number: 02256244.1
(22) Date of filing: 10.09.2002
(51) Int. Cl.: A61F 13/475, A61F 13/472

(54) **Absorbent article**

(30) Priority: 17.09.2001 JP 2001281448
(71) Applicant: Uni-Charm Corporation, Kawanoe-shi, Ehime-ken 182 (JP)
(72) Inventor: Tagami, Etsuko, c/o Technical Center, Mitoyo-gun, Kagawa-ken 769-1602 (JP); Nozaki, Satoshi, c/o Technical Center, Mitoyo-gun, Kagawa-ken 769-1602 (JP)
(74) Representative: Parry, Christopher Stephen

(57) **Abstract**

Disclosed is an absorbent article including: a main body portion having longitudinally opposed front and rear edges; and a surface structure having an ability to absorb and retain a liquid. The surface structure extends longitudinally of the main body portion and is fixed on a body surface of the main body portion through a bond. The bond extends longitudinally of the main body portion to have longitudinally opposed front and rear edges. The surface structure is longitudinally sectionalized into a front portion having the bond therein and a rear free portion extending beyond the rear edge of the bond toward the rear edge of the main body portion. A width of the rear free portion is smaller than a width of the main body portion and also smaller than a width of the front portion of the surface structure.

## Description

### BACKGROUND OF THE INVENTION

### Field of the Invention

The present invention relates to an absorbent article such as sanitary napkin, urine-absorbing pad or the like, more particularly, relates to an absorbent article in which a rear portion of an absorbent member can be moved to follow the motion of the wearer's body so as to improve fit.

### Description of the Related Art

There has been known an absorbent article of which a rear region is adapted to fit with the wearer's body so as to prevent a discharged liquid from leaking along the wearer's buttocks.

In Japanese Unexamined Patent Publication No. H11-104169 (104169/1999), there is disclosed a substantially elongated absorbent article having an absorbent layer for liquid retention disposed on a liquid-impermeable, leakage preventing layer. In this absorbent article, the absorbent layer is fixed at the center of the length to the leakage preventing layer, and the absorbent layer is provided with a rear free edge not fixed to the leakage preventing layer. Moreover, the absorbent layer is provided with longitudinally extending elastic members, so that the rear free edge is raised upwardly from the leakage preventing layer due to contraction of the elastic members.

Upon wearing the absorbent article, the back side of the leakage preventing layer is attached to a short panty through a pressure sensitive adhesive. At this time, even if the short panty is loose or the wearer's body moves during wear, the rear free edge of the absorbent layer can easily be curved to fit with the back part of the wearer's body. As a result, the fit can be improved and the liquid leakage can be effectively prevented.

In the absorbent article disclosed in the above-mentioned publication, however, the width of the absorbent layer is constant from its front edge to the rear free edge or gradually increased toward the rear free edge. Therefore, when worn by a female during menstruation, although the rear portion of the absorbent layer rising from the leakage preventing layer can fit with the anus and the surface of buttocks, it is still difficult to eliminate the space between the gluteal fold and the absorbent layer. Accordingly, a discharged menstrual blood readily flows along the anus and gluteal fold to reach the back part of the body, so that the discharged liquid may possibly spread over a large area of the surface of the absorbent layer, resulting in easily leaking out of the absorbent article.

When the wearer walks or crosses her legs in a sitting or side-lying position, on the other hand, the gluteal fold is deformed to incline rightward or leftward to the body axis. In the absorbent article disclosed in the above-mentioned publication, however, since the rear portion of the absorbent layer covering the anus and the gluteal fold is not freely movable rightward or leftward upon twist of the body, the space between the gluteal fold and the absorbent layer is increased more when the wearer's body is in such positions, to thereby increase the possibility of the liquid leakage. When the wearer's body is in such position, additionally, the short panty is deformed to follow the motion of the flesh of the buttocks, so that the absorbent article attached to the crotch portion of the short panty is easily twisted. This also results in increasing the space between the absorbent layer and the gluteal fold.

The above-mentioned publication also discloses a raised portion formed in the rear portion of the absorbent layer. However, since the rear portion of the absorbent layer is not freely deformable rightward or leftward, when the body is in such positions to deform the gluteal fold, the raised portion cannot easily follow the change of the gluteal fold. Therefore, the raised portion is liable to get out of the gluteal fold, so that improvement of the fit is limited.

### SUMMARY OF THE INVENTION

The present invention has been worked out in view of the shortcoming in the prior art set forth above. It is therefore an object of the present invention to provide an absorbent article in which a rear portion can easily fit in the wearer's gluteal fold and such fit in the gluteal fold can easily be maintained even if the wearer's body moves, so as to improve the effect of preventing leakage of discharged liquid.

According to the present invention, there is provided an absorbent article comprising:
a main body portion having longitudinally opposed front and rear edges and transversely opposed side edges; and
a surface structure having an ability to absorb and retain a liquid, the surface structure extending longitudinally of the main body portion and being fixed on a body surface of the main body portion through a bond, the bond extending longitudinally of the main body portion to have longitudinally opposed front and rear edges, wherein
the surface structure is longitudinally sectionalized into a front portion having the bond therein and a rear free portion extending beyond the rear edge of the bond toward the rear edge of the main body portion, wherein
a width of the rear free portion is smaller than a width of the main body portion and also smaller than a width of the front portion of the surface structure.

Preferably, the rear free portion is gradually narrowed toward a rear edge thereof.

In the absorbent article of the present invention, the surface structure having the ability to absorb and retain a liquid is partly fixed on the body surface of the main body portion. In addition, the surface structure has the rear free portion of a relatively small width. When the absorbent article is worn, therefore, the rear free portion of the surface structure can easily fit with the anus and gluteal fold. Even if the gluteal fold is moved leftward or rightward due to the position of the wearer's body, moreover, the rear free portion of the surface structure can easily follow the motion of the gluteal fold, so that the fit of the rear free portion with the gluteal fold can be maintained to improve the effect of preventing leakage.

Preferably, the surface structure is formed with a raised portion, which is raised along a longitudinally extending centerline of the main body portion, continuously over the front portion and the rear free portion. In the case where the surface structure is formed with such raised portion, since the raised portion can fit with the wearer's body from the female genital organ, through the anus, to the gluteal fold, a discharged liquid can easily be collected by the surface structure to inhibit liquid leakage more effectively. In this case, a height from a garment surface of the main body portion to a top of the raised portion is preferably larger in the rear free portion than in the front portion. By making the rear portion of the raised portion higher than the front portion of the raised portion, the rear portion of the raised portion can easily be fitted into the gluteal fold, while the front portion of the raised portion is kept in close contact with the female genital organ.

It is also possible that the front portion of the surface structure is formed with side free portions which are extended transversely outwardly beyond transversely opposed side edges of the bond. In this case, since the side free portions of the surface structure are hardly moved away from the wearer's body even if the main body portion fixed on an undergarment is twisted by the motion of the wearer's body, transverse leakage of discharged liquid can easily be prevented.

Preferably, the main body portion is longitudinally sectionalized into a front region and a rear region having the entire rear free portion of the surface structure provided therein, wherein a width of the rear region is larger than a width of the front region. More preferably, the main body portion is provided at least in the rear region with an absorbent layer, which is extended beyond a periphery of a region on which the rear free portion is laid. By making the rear region of the main body portion wider, as set forth above, the rear region of the main body portion can cover a large area of the wearer's body including the anus and gluteal fold upon use, while having the rear free portion of the surface structure fitting in the gluteal fold. Therefore, rearward leakage of liquid can be certainly prevented.

Preferably, the rear free portion is transversely deformable to such an extent that a line extending between a center of the rear edge of the bond and a center of a rear edge of the rear free portion is allowed to incline at equal to or more than 5 degrees to a longitudinally extending centerline of the main body portion. With such construction, even if a wearer walks or crosses her legs in sitting or side-lying position during wear, the rear free portion can follow the deformation of the gluteal fold. Accordingly, the fit of the rear free portion with the gluteal fold can easily be maintained to improve the effect of preventing liquid leakage toward the buttocks.

Preferably, when a pressure of 4,900 Pa is applied to the rear free portion from above, a compressive recovery is equal to or more than 30% in a dry state. Also preferably, when a pressure of 4,900 Pa is applied to the rear free portion from side, a compressive recovery is equal to or more than 30% in a dry state.

Preferably, when a pressure of 4,900 Pa is applied to the rear free portion from above, a compressive stiffness is equal to or less than 0.6 in a dry state. Also preferably, when a pressure of 4,900 Pa is applied to the rear free portion from side, a compressive stiffness is equal to or less than 0.6 in a dry state.

With the compressive stiffnesses of the rear free portion being set within the above-mentioned range, the rear free portion can be flexibly deformed to conform to the shape of the gluteal fold. With the compressive recoveries being set within the above-mentioned range, on the other hand, the rear free portion can fit in the gluteal fold with an appropriate elasticity, so that the fit of the rear free portion with the gluteal fold can be constantly maintained.

### BRIEF DESCRIPTION OF THE DRAWINGS

The present invention will be understood more fully from the detailed description given hereinafter and from the accompanying drawings of the preferred embodiment of the present invention, which, however, should not be taken to be limitative to the invention, but are for explanation and understanding only.

In the drawings:
Fig. 1 is a top plan view showing a sanitary napkin as an absorbent article according to one embodiment of the present invention;
Fig. 2 is a sectional view taken along line II - II of Fig. 1;
Fig. 3 is a sectional view taken along line III - III of Fig. 1;
Fig. 4 is a sectional view taken along line IV - IV of Fig. 1;
Fig. 5 is a top plan view illustrating an area where a bond is formed; and
Fig. 6 is a diagram showing compression properties.

### DESCRIPTION OF THE PREFERRED EMBODIMENT

The present invention will be discussed hereinafter in detail in terms of the preferred embodiment according to the present invention with reference to the accompanying drawings. In the following description, numerous specific details are set forth in order to provide a thorough understanding of the present invention. It will be obvious, however, to those skilled in the art that the present invention may be practiced without these specific details. In other instance, well-known structures are not shown in detail in order to avoid unnecessary obscurity of the present invention.

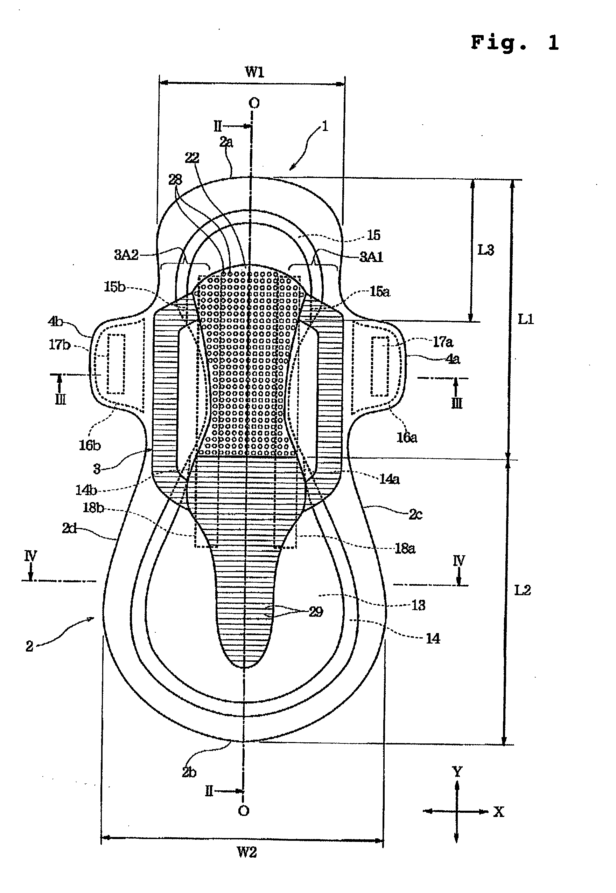

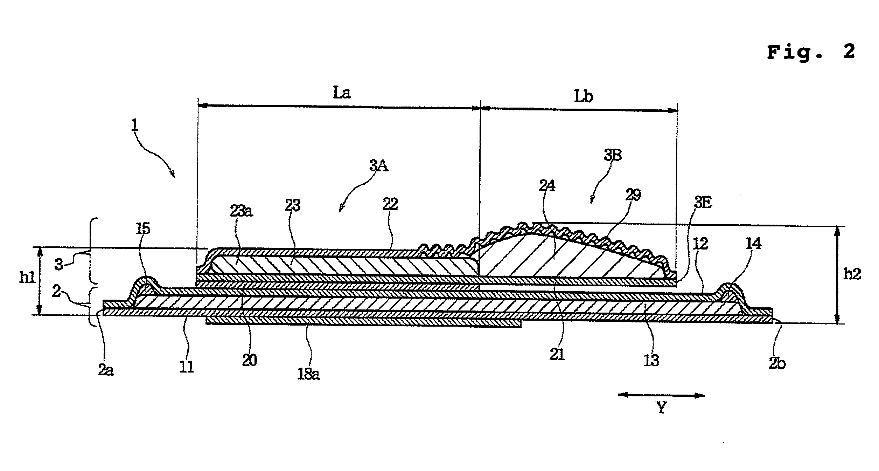

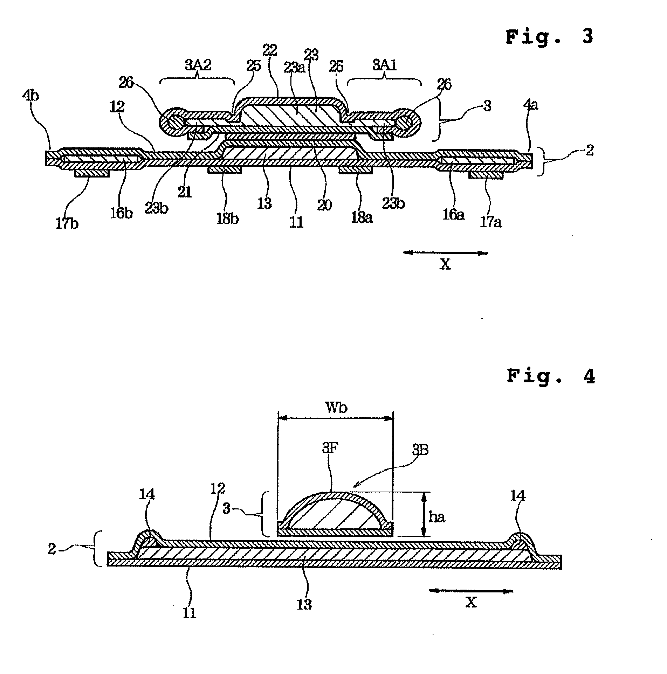

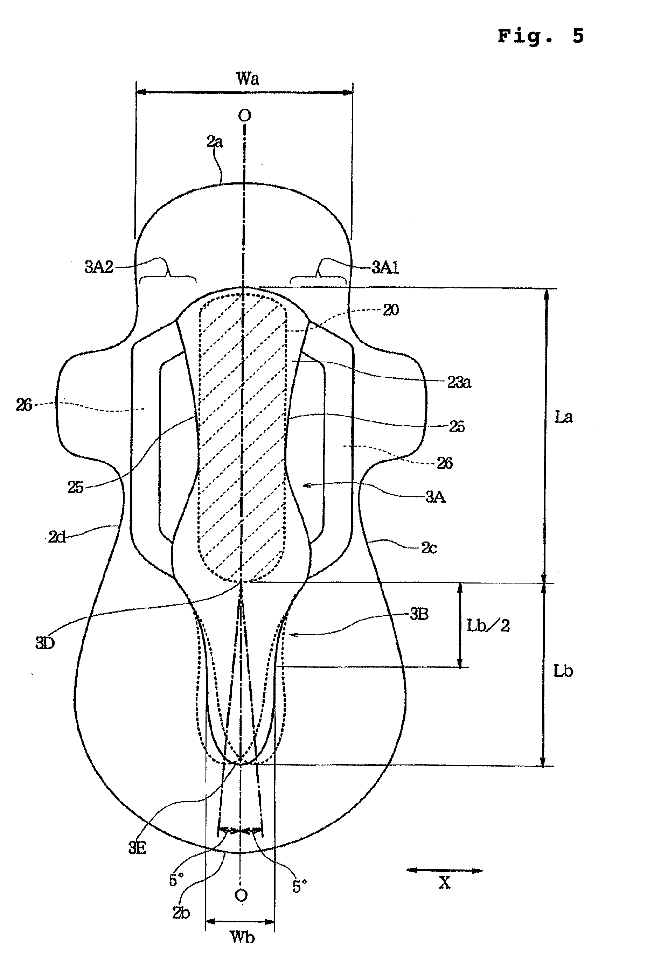

Fig. 1 is a top plan view of a sanitary napkin 1 as an absorbent article according to one embodiment of the present invention; Fig. 2 is a sectional view taken along line II - II of Fig. 1; Fig. 3 is a sectional view taken along line III - III of Fig. 1; and Fig. 4 is a sectional view taken along line IV - IV of Fig. 1. In addition, Fig. 5 is a top plan view illustrating an area where a bond is formed.

The sanitary napkin 1 shown in Figs. 1 to 3 is to be worn by a female during menstruation while being attached to an inner side of a crotch portion of an undergarment (e.g., short panty). The sanitary napkin 1 is constructed to include a main body portion 2 and a surface structure 3 partly fixed on a body surface of the main body portion 2. Here, it should be noted that individual components of the sanitary napkin 1 have a body surface and a garment surface. As used herein, " body surface" means that surface of the components which is intended to be worn toward or adjacent to the body of the wearer, while the " garment surface" is on the opposite side and is intended to be worn toward or placed adjacent to the undergarment when the sanitary napkin 1 is worn.

The main body portion 2 is meant to indicate a portion which is to be put on the inner side of the crotch portion of the undergarment during use. Therefore, wing portions 4a and 4b to be folded back against an outer side of the crotch portion during use (which will be discussed later) are not included in the main body portion 2. The main body portion 2 has a top plan shape of approximately arcuate front and rear edges 2a and 2b and right and left side edges 2c and 2d. The main body portion 2 is elongated in the Y-direction. The main body portion 2 can be sectionalized (divided) into a front region of a length L1 and a rear region of a length L2. The lengths L1 and L2 are almost equal to each other so that the boundary between the front and rear regions extends near and along a transversely extending centerline of the main body portion 2. The front region of the main body portion 2 has a width W1 between the right and left side edges 2c and 2d, and the rear region of the main body portion 2 has a width W2 between the right and left side edges 2c and 2d, wherein the width W2 of the rear region is larger than the width W1 of the front region.

Throughout this disclosure, unless otherwise noted, the term " length" means a maximum dimension of each component/portion/region as measured in the longitudinal direction (Y-direction) of the main body portion 2. Similarly, the term " width" means a maximum dimension of each component/portion/region as measured in the transverse direction (X-direction) of the main body portion 2, and the term " height" means a maximum dimension of each component/portion/region as measured in the direction perpendicular to the X-Y plane.

In the front region, the above-mentioned wing portions 4a and 4b are formed to protrude transversely outwardly from the right and left side edges 2c and 2d of the main body portion 2, respectively.

As shown in Figs. 2 to 4, the main body portion 2 comprises a liquid impermeable backsheet 11, a liquid permeable topsheet 12, and an absorbent layer 13 disposed between the backsheet 11 and the topsheet 12 for absorbing a liquid.

In the rear region of the length L2, moreover, a leakage preventing layer 14 is laid on the absorbent layer 13, along the periphery of the absorbent layer 13, and covered with the topsheet 12. The leakage preventing layer 14 has an ability to absorb and retain a liquid. In the rear region, the leakage preventing layer 14 extends continuously along the periphery of the absorbent layer 13 and terminates at 14a and 14b, as shown in Fig. 1. In the front region of the length L1, on the other hand, a leakage preventing layer 15 similar to the leakage preventing layer 14 is provided on the absorbent layer 13, along the periphery of the absorbent layer 13, and covered with the topsheet 12. In the front region, however, the leakage preventing layer 15 terminates at 15a and 15b, as shown in Fig. 1, so that the leakage preventing layer 15 is provided only within a region having a length L3 which is about half the length L1.

Outside the absorbent layer 13 and the leakage preventing layers 14 and 15, the backsheet 11 and the topsheet 12 are joined to each other through a hot-melt type adhesive.

In the shown embodiment, as shown in Fig. 1, the leakage preventing layers 14 and 15 are provided without overlapping with the surface structure 3, except for side free portions 3A1 and 3A2 (which will be described later). With the leakage preventing layers 14 and 15 thus provided along the periphery of the absorbent layer 13, therefore, the body surface of the main body portion 2 is protruded to form leakage preventing protrusions. Thus, front and rear liquid-absorbing regions of the main body portion 2, which have the absorbent layer 13 therein but do not overlap with the surface structure 3, are provided along their outward edges with the leakage preventing protrusions.

As shown in Fig. 3, the wing portions 4a and 4b have reinforcing papers 16a and 16b, respectively, between the backsheet 11 and the topsheet 12. The backsheet 11, the individual reinforcing papers 16a and 16b and the topsheet 12 thus stacked are fixed through a hot-melt type adhesive. With the reinforcing papers 16a and 16b, the stiffness of the wing portions 4a and 4b can be increased, so that the wing portions 4a and 4b can easily be folded back against the garment surface of the main body portion 2 upon attachment to the undergarment.

As shown in Figs. 1 and 3, moreover, the wing portions 4a and 4b are provided on the exterior surface of the backsheet 11 with pressure sensitive adhesive layers 17a and 17b functioning as fixing means, respectively. On the other hand, the main body portion 2 is provided on the exterior surface of the backsheet 11 with pressure sensitive adhesive layers 18a and 18b, which are in the shape of strips extending longitudinally in a parallel relationship with each other.

The surface structure 3 is partly bonded to the body surface of the main body portion 2 through a hot-melt type adhesive or the like. In Figs. 2, 3 and 5, such bond is indicated at 20.

The surface structure 3 is longitudinally sectionalized (divided) into a front portion 3A and a rear free portion 3B. In the shown embodiment, the front portion 3A has the bond 20 therein, and the rear free portion 3B extends beyond the rear edge of the bond 20 toward the rear edge 2b of the main body portion 2 (i.e., rearwardly).

In the front portion 3A, moreover, the surface structure 3 is. protruded transversely outwardly beyond the side edges of the bond 20, to thereby form the above-mentioned side free portions 3A1 and 3A2, as part of the front portion 3A.

As shown in Fig. 2, the surface structure 3 comprises a backsheet 21 and a topsheet 22. Between the backsheet 21 and the topsheet 22, a first absorbent layer 23 is provided in the front portion 3A and a second absorbent layer 24 is provided in the rear free portion 3B.

As shown in Fig. 3, the front portion 3A of the surface structure 3 is formed with compressed grooves 25 and 25, which are spaced equally transversely apart from a longitudinally extending centerline O - O. Between the compressed grooves 25 and 25, the first absorbent layer 23 is of a large thickness to provide a raised portion 23a. Transversely outside the compressed grooves 25 and 25, on the other hand, the thickness of the first absorbent layer 23 is made slightly smaller than that of the raised portion 23a, to provide thin portions 23b and 23b. Moreover, the front portion 3A of the surface structure 3 is provided along the periphery of the absorbent layer 23 (along the outward edges of the thin portions 23b and 23b) with leakage preventing layers 26 and 26. These leakage preventing layers 26 and 26 have an ability to absorb and retain a liquid similar to the first absorbent layer 23, and have a larger thickness than the thin portions 23b and 23b.

Thus, in the first portion 3A of the surface structure 3 having the first absorbent layer 23 and the leakage preventing layers 26 and 26 disposed between the backsheet 21 and the topsheet 22, the thickness of the first portion 3A is increased near the outward edges of the side free portions 3A1 and 3A2 with the body surface of the first portion 3A upwardly protruded by the leakage preventing layers 26 and 26, as well as in the raised portion 23a.

In the rear free portion 3B of the surface structure 3, on the other hand, the second absorbent layer 24 is disposed between the backsheet 21 and the topsheet 22. As shown in Figs. 1 and 5, the rear free portion 3B is continued from the raised portion 23a of the front portion 3A, so that the raised portion 23a and the rear free portion 3B form a continuous raised portion along the centerline O - O.

Here, as shown in Fig. 2, the height h2 from the backsheet 11 to the top of the rear free portion 3B is larger than the height h1 from the backsheet 11 to the top of the raised portion 23a of the front portion 3A.

The topsheet 22 is formed of a liquid permeable nonwoven fabric. As shown in Fig. 1, a portion of the topsheet 22, which covers most of the raised portion 23a within the front region of the main body portion 2, is formed with a large number of liquid passage holes 28; and a portion of the topsheet 22, which covers the rear free portion 3B and the remaining part of the raised portion 23a within the rear region of the main body portion 2, is formed with corrugations (wrinkles) 29. Such corrugations are also formed along the outward edges of the side free portions 3A1 and 3A2.

The backsheet 21 is preferably formed of a liquid impermeable sheet. In the case where the absorbent layer 13 is provided in the main body portion 2, as in the shown embodiment, the backsheet 21 may be permeable to liquid. If such absorbent layer 13 is not provided in the main body portion 2, however, it is required to form the backsheet 21 of a liquid impermeable sheet.

The first absorbent layer 23 and the second absorbent layer 24 may be formed of the same material. However, it is preferred that the second absorbent layer 24 has a higher compressive recovery and a lower compressive stiffness than the raised portion 23a of the first absorbent layer 23.

The backsheet 11 of the main body portion 2 is formed of a liquid impermeable sheet, such as polyethylene (PE) film or laminate sheet of the PE film and nonwoven fabric. On the other hand, the topsheet 12 is permeable to liquid, as exemplified by through-air bonded nonwoven fabric of which synthetic fibers are thermally bonded with hot air, spunlaced nonwoven fabric of which synthetic fibers are hydroentangled, or resin film having liquid passage holes. Such nonwoven fabrics may also be formed with liquid passage holes.

The topsheet 22 of the surface structure 3 may also be formed of such through-air bonded or spunlaced nonwoven fabric. Since the front portion 3A of the surface structure 3 comes into contact with the vaginal opening of a female during use to function as a portion for mainly receiving a discharged liquid, the topsheet 22 is preferably formed at such portion with the liquid passage holes 28. On the other hand, since the rear free portion 3B comes into contact mainly with the anus and gluteal fold during use, the topsheet 22 is preferably formed at the rear free portion 3B with the corrugations 29, so as to provide soft contact feel.

In the sanitary napkin 1, the length (L1 + L2) of the main body portion 2 is in a range of 200 to 450 mm. Assuming that the sanitary napkin 1 is to be used during bedtime, the length of the main body portion 2 is preferably equal to or more than 250 mm. If the main body portion 2 is too long, there may be a possibility of giving an unpleasant feeling to the buttocks during wear. Therefore, the length of the main body portion 2 is preferably in a range of 250 to 350 mm. On the other hand, the width W1 of the front region of the main body portion 2 is in a range of 40 to 100 mm; the width W2 of the rear region is in a range of 80 to 150 mm.

As shown in Fig. 5, the rear edge of the bond 20 functions as a boundary 3D between the front portion 3A and the rear free portion 3B of the surface structure 3. The length (La + Lb) of the surface structure 3 is in a range of about 80 to 300 mm. The distance between the front edge 2a of the main body portion 2 and the boundary 3D is in a range of 130 to 200 mm, and the length Lb of the rear free portion 3B is in a range of 30 to 120 mm.

The width of the rear free portion 3B of the surface structure 3 is smaller than that of the front portion 3A, and the rear free portion 3B is gradually narrowed from the boundary 3D toward the rear edge 3E. In order to come into close contact with the anus and gluteal fold of a wearer, the rear free portion 3B of the surface structure 3 should be relatively narrow. More specifically, the width Wb of the rear free portion 3B at the center of the length Lb between the boundary 3D and the rear edge 3E (at the position spaced apart from the boundary 3D by Lb/2) is preferably equal to or less than 2/3, more preferably equal to or less than 1/2 the width Wa of the front portion 3A. In order to further facilitate fit into the gluteal fold, the width Wb is preferably equal to or less than 50 mm, more preferably equal to or less than 30 mm.

Although the rear free portion 3B is continued from the raised portion 23a of the front portion 3A, it is preferred that the sectional shape of the rear free portion 3B is not flat but protruded to have a top 3F, as shown in Fig. 4. At the position spaced apart from the boundary 3D by Lb/2, moreover, the ratio of the height ha (from the garment surface of the rear free portion 3B to the top 3F) to the width Wb, i.e., the ratio of ha/Wb is preferably equal to or more than 0.2, more preferably equal to or more than 0.3. The rear free portion 3B thus protruded upwardly can easily fit with the anus and gluteal fold.

In order to facilitate the fit of the rear free portion 3B with the anus and gluteal fold and to maintain the fit in conformity with the motion of the wearer's body, moreover, the rear free portion 3B preferably has the following properties.

Preferably, the rear free portion 3B is transversely deformable to such an extent that, when the rear edge 3E is pulled or pushed in the transverse direction (X-direction) of the sanitary napkin 1, a line extending between the center of the boundary 3D (i.e., the center of the rear edge of the bond 20) and the center of the rear edge 3E of the rear free portion 3B is allowed to incline at equal to or more than 5 degrees to the longitudinally extending centerline O - O of the main body portion 2, as indicated by dotted line in Fig. 5. Here, the center of the boundary 3D refers to the intersection of the boundary 3D and the centerline O - O when the rear free portion 3B is not transversely deformed, and the center of the rear edge 3E refers to the intersection of the rear edge 3E and the centerline O - O when the rear free portion 3B is not transversely deformed. It is also preferred that a force required to be applied to the rear edge 3E in the transverse direction (X-direction) for deforming the rear free portion 3B to incline at 5 degrees is equal to or less than 0.2 N.

The measurement of such force can be performed as follows. A hook is attached to a contact of Digital Force Gauge " SHIMPO FGC-0.2" manufactured by NIDEC-SHIMPO CORPORATION, Japan, and then put on the rear edge 3E of the rear free portion 3B. Thereafter, the rear edge 3E is transversely pulled until the rear free portion 3B is deformed to incline at 5 degrees, and at this time, the load displayed by the digital force gauge is read.

If the rear free portion 3B can be transversely deformed to incline at 5 degrees or more and the force required to deform the rear free portion 3B to incline at 5 degrees is 0.2 N or less, the rear free portion 3B can readily be deformed to follow the motion of the gluteal fold during wear, so that the fit of the rear free portion 3B with the gluteal fold can easily be maintained.

On the other hand, the compression properties of the rear free portions 3B are preferably as follows.

Throughout this disclosure, the compressive recovery and compressive stiffness of the rear free portion 3B are expressed by values determined by using Automatic Compression Tester " KES FB3-A" manufactured by Kato Tech, Japan.

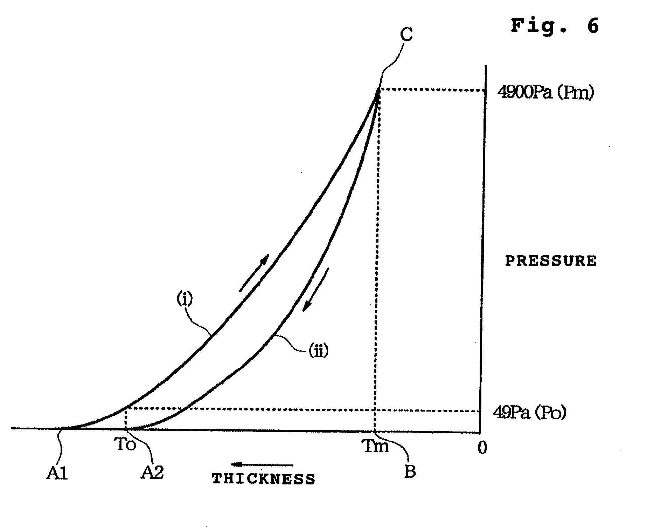

For measurement of such values, a test sample of the same structure and size as the rear free portion 3B is prepared, put on a measuring platform of the Automatic Compression Tester, and then, pressed with a pressure plate (circular pressure plate having an area of 2 cm²). In the diagram of Fig. 6, the applied pressure is plotted in ordinate, and the thickness of the test sample is plotted in abscissa. The thickness of the pressed region of the test sample, when an initial pressure of P0 = 49 Pa (0.5 g/cm²) is applied to the test sample with the pressure plate, is designated initial thickness T0. Then, the compression pressure is increased linearly at a compression rate of 0.1 cm/second from the initial pressure P0 to the maximum compression pressure of Pm = 4,900 Pa (50 g/cm²). Tm represents the thickness of the pressed region of the test sample when the maximum compression pressure Pm is applied thereto.

The compression energy WC per 1 cm² of the test sample is expressed by a value of the definite integral along the curve (i) of Fig. 6 between Tm and T0, i.e., WC = ∫P · dT (P indicates pressure; T indicates thickness). The compressive stiffness (LC value) is obtained according to LC = 2WC/{(T0 - Tm)Pm} (dimensionless).

On the other hand, the compressive recovery (RC value) is obtained according to RC = R/WC x 100 (%), wherein R is a value of the definite integral along the recovery curve (ii) between Tm and T0, i.e., R = ∫P · dT.

That is, the compressive stiffness is expressed by (WC/the area of the triangle with apexes A1, B and C), wherein WC refers to the area of the region defined by the curve (i), A1, B and C shown in Fig. 6. On the other hand, the compressive recovery is expressed by the area ratio of R/WC x 100 %, wherein R refers to the area defined by the curve (ii), A2, B and C.

Such measurement is carried out on the test sample, which has the same structure as the rear free portion 3B and is in a dry state, for both of the case where the pressure is applied from above (downwardly to the top 3F) and the case where the pressure is applied from side (transversely). In both cases, the measured compressive recovery is preferably equal to or more than 30%.

With the compressive recovery being equal to or more than 30%, even if the rear free portion 3B fitting in the gluteal fold is crushed by the pressure from the fold, its shape can easily be restored.

On the other hand, the compressive stiffness of the rear free portion 3B is preferably equal to or less than 0.6 in both of the case where the pressure is applied from above and the case where the pressure is applied from side.

In case where the compressive stiffness is equal to or less than 0.6 even in a dry state, the rear free portion 3B fitting with the anus and gluteal fold hardly gives a stiff feel to a wearer, to be comfortable to wear.

When the sanitary napkin 1 is to be worn, the pressure sensitive adhesive layers 18a and 18b provided on the garment surface of the main body portion 2 are adhered to the inner side of the crotch portion of the undergarment. Then, the wing portions 4a and 4b are folded back against the garment surface of the main body portion 2, and fixed on the outer side of the crotch portion through the pressure sensitive adhesive layers 17a and 17b. After the sanitary napkin 1 is thus fixed on the crotch portion of the undergarment, the undergarment is worn.

At this time, the raised portion 23a of the front portion 3A of the surface structure 3 comes into close contact with the vaginal opening, and the narrow, rear free portion 3B comes into close contact with the anus and gluteal fold.

A menstrual blood discharged from the vaginal opening passes through the liquid passage holes 28 of the topsheet 22 of the surface structure 3, and is then absorbed by the first absorbent layer 23. Since the rear free portion 3B of the surface structure 3 is in close contact with the anus and gluteal fold, on the other hand, the space between the surface structure 3 and the gluteal fold can be eliminated, to thereby prevent rearward leakage of the menstrual blood.

Moreover, the rear free portion 3B has a degree of freedom so as to be transversely deformable. Therefore, even if the wearer's gluteal fold is deformed to incline to the body axis by walking or crossing her legs in sitting or side-lying position, for instance, the rear free portion 3B can be deformed to follow the gluteal fold. Accordingly, the fit of the rear free portion 3B with the gluteal fold can easily be maintained, to constantly prevent rearward leakage of the menstrual blood.

On the other hand, the front portion 3A of the surface structure 3 has the side free portions 3A1 and 3A2 extending transversely outwardly from the raised portion 23a. Therefore, even if the main body portion 2 is inclined to the crotch of the wearer' s body due to looseness or twist of the crotch portion of the undergarment, the side free portions 3A1 and 3A2 can be kept in contact with the vaginal opening or left and right side portions of the vaginal opening in the crotch. Moreover, since the side free portion 3A1 and 3A2 are provided at their outward edges with the leakage preventing layers 26 and 26 to thereby protrude upwardly, a menstrual blood trying to flow beyond the outward edges of the side free portions 3A1 and 3A2 can be dammed up by such protrusions. Still moreover, since the side free portions 3A1 and 3A2 can be kept in contact with the wearer's groin due to the leakage preventing layers 26 and 26, a menstrual blood flowing along the wearer's body can also be dammed up by the leakage preventing layers 26 and 26.

On the other hand, since the main body portion 2 is widened in its rear region having the rear free portion 3B therein and the absorbent layer 13 is also widened in this rear region, even if a menstrual blood flowing along the gluteal fold reaches the rear free portion 3B and then leaks transversely out of the rear free portion 3B, it can be absorbed by the absorbent layer 13 of a large area. Moreover, since the absorbent layer 13 is provided along its periphery with the leakage preventing layer 14 to thereby protrude upwardly, a menstrual blood trying to leak transversely along the absorbent layer 13 can be dammed up by the leakage preventing layer 14. In the front region of the main body portion 2, too, since the absorbent layer 13 is provided along its periphery with the leakage preventing layer 15, leakage of the menstrual blood can easily be prevented.

It should be noted that the absorbent article of the present invention should not be limited to the sanitary napkin, but may include a urine-absorbing pad to be attached to an inner side of an article, such as undergarment or disposable diaper, to be externally worn upon use, or the like.

As has been described hereinabove, since the surface structure of an ability to absorb a liquid has the rear free portion, which can fit in the wearer's gluteal fold and move to follow the motion of the wearer's body, the absorbent article of the present invention can effectively prevent rearward leakage of a discharged liquid.

Although the present invention has been illustrated and described with respect to exemplary embodiment thereof, it should be understood by those skilled in the art that the foregoing and various other changes, omission and additions may be made therein and thereto, without departing from the spirit and scope of the present invention. Therefore, the present invention should not be understood as limited to the specific embodiment set out above but to include all possible embodiments which can be embodied within a scope encompassed and equivalent thereof with respect to the feature set out in the appended claims.

## Claims

1. An absorbent article comprising:
a main body portion having longitudinally opposed front and rear edges and transversely opposed side edges; and
a surface structure having an ability to absorb and retain a liquid, the surface structure extending longitudinally of the main body portion and being fixed on a body surface of the main body portion through a bond, the bond extending longitudinally of the main body portion to have longitudinally opposed front and rear edges, wherein
the surface structure is longitudinally sectionalized into a front portion having the bond therein and a rear free portion extending beyond the rear edge of the bond toward the rear edge of the main body portion, wherein
a width of the rear free portion is smaller than a width of the main body portion and also smaller than a width of the front portion of the surface structure.

2. The absorbent article as set forth in claim 1, wherein the rear free portion is gradually narrowed toward a rear edge thereof.

3. The absorbent article as set forth in claim 1, wherein the surface structure is formed with a raised portion, which is raised along a longitudinally extending centerline of the main body portion, continuously over the front portion and the rear free portion.

4. The absorbent article as set forth in claim 3, wherein a height from a garment surface of the main body portion to a top of the raised portion is larger in the rear free portion than in the front portion.

5. The absorbent article as set forth in claim 1, wherein the front portion of the surface structure is formed with side free portions which are extended transversely outwardly beyond transversely opposed side edges of the bond.

6. The absorbent article as set forth in claim 1, wherein the main body portion is longitudinally sectionalized into a front region and a rear region having the entire rear free portion of the surface structure provided therein, wherein a width of the rear region is larger than a width of the front region.

7. The absorbent article as set forth in claim 6, wherein the main body portion is provided at least in the rear region with an absorbent layer, which is extended beyond a periphery of a region on which the rear free portion is laid.

8. The absorbent article as set forth in claim 1, wherein the rear free portion is transversely deformable to such an extent that a line extending between a center of the rear edge of the bond and a center of a rear edge of the rear free portion is allowed to incline at equal to or more than 5 degrees to a longitudinally extending centerline of the main body portion.

9. The absorbent article as set forth in claim 1, wherein when a pressure of 4,900 Pa is applied to the rear free portion from above, a compressive recovery is equal to or more than 30% in a dry state.

10. The absorbent article as set forth in claim 1, wherein when a pressure of 4,900 Pa is applied to the rear free portion from side, a compressive recovery is equal to or more than 30% in a dry state.

11. The absorbent article as set forth in claim 1, wherein when a pressure of 4,900 Pa is applied to the rear free portion from above, a compressive stiffness is equal to or less than 0.6 in a dry state.

12. The absorbent article as set forth in claim 1, wherein when a pressure of 4,900 Pa is applied to the rear free portion from side, a compressive stiffness is equal to or less than 0.6 in a dry state.
